Europäisches Patentamt

European Patent Office

Office européen des brevets

(19)

(11) Publication number: **0 174 704 B1**

(12) **EUROPEAN PATENT SPECIFICATION**

(45) Date of publication of patent specification: **11.01.89**

(51) Int. Cl.⁴: **A 61 K 7/26,** A 61 K 37/02

(21) Application number: 85201455.4

(22) Date of filing: **11.09.85**

(54) Composition for protecting teeth against decalcification.

(30) Priority: 12.09.84 NL 8402786

(43) Date of publication of application:
19.03.86 Bulletin 86/12

(45) Publication of the grant of the patent:
11.01.89 Bulletin 89/02

(84) Designated Contracting States:
AT BE CH DE FR GB IT LI LU NL SE

(56) References cited:
WO-A-81/02977
NL-C- 69 163

JOURNAL OF ORAL PATHOLOGY, vol. 11, no. 1, 1982, pages 1-17, Munksgaard, Copenhagen, DK; L.A. TABAK et al.: "Role of salivary mucins in the protection of the oral cavity"
CARBOHYDRATE RESEARCH, vol. 108, no. 1, 1982, pages 111-122, Elsevier Scientific Publishing Co., Amsterdam, NL; A. PRAKOBHOL et al.: "Purification of low-molecular-weight, mucin-type glycoprotein from human submandibular-sublingual saliva"

(73) Proprietor: **Stichting Biomaterials Science Center, VU, "BSC-VU"**
De Boelelaan 1115
NL-1081 HV Amsterdam (NL)

(72) Inventor: **de Groot, Klaas**
L. van Wijkplein 6
NL-2101 EL Heemstede (NL)
Inventor: **Roukema, Pieter Andries**
Heijermanslaan 29
NL-1422 GT Uithoorn (NL)
Inventor: **van Nieuw Amerongen, Arie**
G. van Nijenrodestraat 136
NL-3621 GM Breukelen (NL)

(74) Representative: **Urbanus, Henricus Maria, Ir.**
et al
c/o Vereenigde Octrooibureaux Nieuwe Parklaan 107
NL-2587 BP 's-Gravenhage (NL)

Courier Press, Leamington Spa, England.

## EP 0 174 704 B1

(56) References cited:

CHEMICAL ABSTRACTS, vol. 99, 1983, page 99, no. 187710r, Columbus, Ohio, US; T.B.F.M. GELHARD et al.: "Remineralization of softened human enamel in mucin- or CMC-containing artificial salivas", & J. ORAL PATHOL. 1983, 12(5), 336-41

CHEMICAL ABSTRACTS, vol. 95, 1981, page 144, no. 36228f, Columbus, Ohio, US; L.G. SIMONSON et al.: "Effect of human saliva and various compounds on the adsorption on the bacterium Streptococcus mutans to hydroxyapatite", & ARCH. ORAL BIOL. 1981. 26(2), 143-6

CHEMICAL ABSTRACTS, vol. 96, 1982, page 359, no. 213674s, Columbus, Ohio, US; P.A. ROUKEMA et al.: "Ultracentrifugation of salivary mucins", & ADV. EXP. MED. BIOL. 1982, 144(Mucus Health Dis.-2), 179-81 "Glycoproteins. Their composition, structure and function", 2nd edition, part B, vol. 5, 1972, pages 810-829, Elsevier Publishing Co., Amsterdam, NL; A. GOTTSCHALK et al.: "Submaxillary gland glycoproteins"

**Description**

This invention relates to a composition for protecting teeth against decalcification induced by the action of acid.

One of the problems regularly encountered in dentistry is the acid-action-induced decalcification of teeth.

It is an object of the present invention to provide a contribution towards solving this problem.

This object is realized by a composition which, according to the present invention, is characterized by containing mucins isolated from saliva secreted by human mucous salivary glands, or synthetic analogues of human mucins.

Preferred embodiments of the composition according to the present invention are toothpastes and artificial salivas.

It has surprisingly been found that human salivary mucins not only, as compared to animal mucins, are exceedingly strongly adsorbed to hydroxyapatite (HAP) and, in this, are hardly, if at all, hindered by the presence of phosphate-containing proteins, but also give an exceedingly good protection of dental enamel against acid action by virtue of the fact that, unlike proteins present in the parotid saliva with a comparably strong adsorption to HAP, such as statherine and proline-rich proteins, they prevent acid-action-induced decalcification of teeth.

The old Netherlands patent 69163 discloses dentifrices, such as toothpastes and mouthwaters containing (extracts of) salivary glands in connection with their favourable effect on teeth, but does not show the insight that mucins separated from human saliva have particular properties, namely, a much stronger adsorption to hydroxyapatite than animal mucins, which adsorption is in addition much less sensitive to competition with phosphate-containing proteins, and an excellent protection of dental enamel against acid attack. The above Dutch patent only describes the use of animal, for example bovine, salivary glands (or extracts thereof).

The PCT application WO81/02977 is slightly more relevant in using the word mucins, but is also limited to non-human material (claim 1: 'non-human mammalian mucin') such as PGM (porcine gastric mucin) and BSM (bovine salivary mucin).

The article by Prakobphol et al. in Carbohydrate Research 108 (1982), 111—122 relates to a human submandibular sublingual salivary mucin, but gives no indications that this human material could be a suitable component of compositions for protecting teeth against acid-induced decalcification and is much better suitable for this purpose than are animal mucins.

Chemical Abstracts 99:187710r (1983) contains no data about the origin of the mucin used in the experiments: in addition, the results appear to be indicative of a preference for carboxymethyl cellulose to mucin as a component of artificial saliva.

Chemical Abstracts 95:36228f (1981) contains no suggestion whatsoever to use specifically human mucin as a component of teeth protecting compositions.

An article by the present inventors in Adv. Exp. Med. Biol. 1982 144, 179—181 (Chemical Abstracts 96:213674s) gives a comparison between some physico-chemical properties of several mucins (HSL, MSL, HSM, MSM), but does not disclose the essence of the present invention, namely, the surprising advantages of human mucins over and above animal mucins in protecting teeth against acid-induced decalcification.

In the following, more detailed, description of the invention, reference is made to the accompanying Figures 1—5 in which

Figure 1 shows an example of a model of the ovine submandibular mucin (OSM).

Figure 2 shows the most important types of oligosaccharide chains of various kinds of mucins, namely, ovine submandibular mucin (OSM), porcine submandibular mucin (PSM), porcine gastric mucin (PGM) and human salivary mucin (HWSM);

Figure 3 is a graph showing the relationship between the amount of mucin adsorbed to hydroxyapatite (HAP) and the initial mucin concentration for several kinds of mucins;

Figure 4 is a graph showing the relationship between the amount of mucin adsorped to HAP and the initial phytate concentration for several kinds of mucins; and

Figure 5 shows an example of a synthetic mucin-like product.

Human saliva contains various types of polypeptides, including enzymes such as amylase, involved in digestion, proline-rich proteins, involved in the adsorption to dental enamel and maintaining the supersaturation of saliva with $Ca^{2+}$ and mucins providing for the viscosity of saliva.

The mucins form a group of biopolymers which are of great importance for maintaining the health of the oral cavity, both the teeth and the epithelial tissue. Salivary mucins are produced and secreted by several kinds of mucous salivary glands, the largest of which are: glandula Submandibularis (SM) and glandula Sublingualis (SL). In addition there are countless minor mucous salivary glands in the epithelial tissue of tongue, palate, cheek and lip, which also secrete mucins. No mucins are produced and secreted, however, by serous salivary glands, such as glandula parotis (Par). This last salivary gland mainly synthesises amylase and proline-rich proteins. Indeed, pure parotidal saliva is not viscous.

The mucins are built up from a long stretched polypeptide chain (500—4000 amino acids), with a short or somewhat longer carbohydrate chain being covalently bonded to the amino acids Serine (Ser) and Threonine (Thr) via N-acetylgalactosamine (GalNac). The mucins are characterized by their terminal sugar:

a. sialic acid (=neuraminic acid), contains a carboxyl group, owing to which they give a negative charge to the mucins: acid mucins.

b. fucose, contains no charged group: neutral mucins.

Both sialo and fucomucins may contain sulphate groups.

The physical and biological properties of a mucin are greatly determined by the length and composition of the polypeptide chain. These determine the number of possible carbohydrate chains. As the carbohydrate chains are highly hydrophilic, the mucins have a large watercoat around them, owing to which they occupy a large volume in aqueous solution. The water coat provides for the mucous properties.

Mucins have various functions in the oral cavity:

— they render the saliva viscous so that it does not flow easily

— they moisten the dental surface and the mucosa and so protect these tissues from drying-up

— they protect the mucosa against bacterial infection by forming a mucous layer which is hard to penetrate by bacteria

— they protect the teeth against decalcification and acid attack; they form a barrier to the $H^+$ ions

— they aggregate certain oral bacteria, thus rendering these harmless.

Isolation of salivary mucins from human saliva

Human mucous saliva is obtained by stimulating secretion by means of a few citric acid pellets on the tongue, while the ductal orifices of the gl. Parotis are covered with a Lashley cup connected to outlet tubes. This results in highly viscous saliva originating especially from the large salivary glands gl. Submandibularis and gl. Sublingualis so that it is designated as SM-SL-saliva. This mucous saliva is collected in an ice cooled glass beaker, containing 30 µl 0.1% PMSF (phenylmethylsulphonic acid fluoride) per 30 ml as an inhibitor of proteolytic decomposition. The saliva is kept at −20°C until sufficient starting material has been collected.

Owing to the high molecular weight of the mucins (500,000—2,000,000 dalton) they can be separated from the globular salivary proteins by means of ultracentrifugation (Roukema et al., Biochim. Biophys. Acta *428*, (1976) 432—440, Roukema and Nieuw Amerongen in: Saliva and Dental Caries (1979) pp. 68—80).

Operations:

— defrost the saliva and add N-acetylcystein to 0.02 M final concentration

— stir with magnetic stirrer for 3 minutes

— centrifuge at 10,000×g for 10 minutes

— dialyse supernatant overnight

— centrifuge retentate at 100,000×g for two hours. Owing to low pH (about 3) the mucins are highly viscous and sediment.

— suspend sediment in a buffer of pH 7.15 consisting of: 0.11 M NaCl+0.014 M $Na_2HPO_4$+0.056 M $NaH_2PO_4$ followed by centrifugation at 100,000×g for 1 hour.

— subsequently centrifuge the clear supernatant at 100,000×g for 24 hours to sediment the mucins.

— suspend the bottoms fraction in a Na acetate buffer pH 4.5, heat it at 100°C for 10 minutes and centrifuge in a tabletop centrifuge.

— transfer the supernatant to the centrifugation buffer pH 7.15 and centrifuge at 100,000×g for 24 hours.

— suspend the sediment in distilled water and extensively dialyse.

The retentate is dry-frozen and contains the total mucin fraction of human saliva, designated by HWSM: Human Whole Salivary Mucins. Using this mucin fraction, the experiments were conducted after testing for the absence of contaminating proteins by means of polyacrylamide gel electrophoresis.

Some biochemical characteristics of HWSM

As stated before, mucins consist of a long polypeptide chain, about which is a dense packing of shorter or longer oligosaccharide chains which protect the polypeptide chain from proteolytic decomposition. Roughly the mucins consist as to 10—30% of protein and as to 70—90% of sugars. HWSM consists as to about 18% of protein by the amino acids assay. The most important amino acids of HWSM are Threonine (14.7%), Valine (12.6%), Serine (10.8%) and Proline (8.4%), see Table A. A carbohydrate chain may be covalently bonded to the hydroxyle group of the Threonine and Serine amino acids via the binding sugar N-acetylgalactosamine (GalNac). The chemical composition of the human salivary mucins is given in Table B.

TABLE A
Acid composition of mucins from human saliva

|  | Number per 100 amino acids |
| --- | --- |
| Asparagic acid | 6.1 |
| Threonine | 14.7 |
| Serine | 10.8 |
| Glutamic acid | 7.3 |
| Proline | 8.4 |
| Glycine | 6.9 |
| Alanine | 7.4 |
| Cysteine | N.D. |
| Valine | 12.6 |
| Methionine | N.D. |
| Isoleucine | 2.0 |
| Leucine | 5.5 |
| Tyrosine | 2.0 |
| Phenylalanine | 2.4 |
| Lysine | 5.1 |
| Histidine | 2.5 |
| Arginine | 6.4 |

Trp was not determined

TABLE B
Chemical composition of human salivary mucins (HWSM) on the basis of the dry weight

|  | Wt.% |  | Molar ratio |
| --- | --- | --- | --- |
| Protein |  |  |  |
| Amino acids | 18.2 |  |  |
| Lowry proteins |  | 14.2 |  |
| Hexoses | 20.4 |  |  |
| Galactose |  | 17.9 | 6.0 |
| Mannose |  | 0.7 | 0.2 |
| Glucose |  | 1.8 | 0.6 |
| Hexosamines | 31.2 |  |  |
| Glucosamine |  | 17.1 | 4.7 |
| Galacotsamine |  | 14.1 | 3.9 |
| Fucose | 17.4 |  | 6.4 |
| Sialic acid | 5.1 |  | 1.0 |
| Sulfate | 1.4 |  | 1.1 |
| Phosphate | 0.14 |  | 0.1 |
| Total | 93.84% |  |  |

The carbohydrate concentration of HWSM is 74% including 5.1% sialic acid and 17.4% fucose as terminal sugars. Sialic acid is of importance for the negative charge, as this sugar contains an ionised $COO^-$ group, unlike fucose. In addition HWSM contains as negatively charged functional groups: sulphate (1.4%) and phosphate (0.14%) and also carboxylic groups of the acid amino acids asparagic acid (6.1%) and glutamic acid (7.3%).

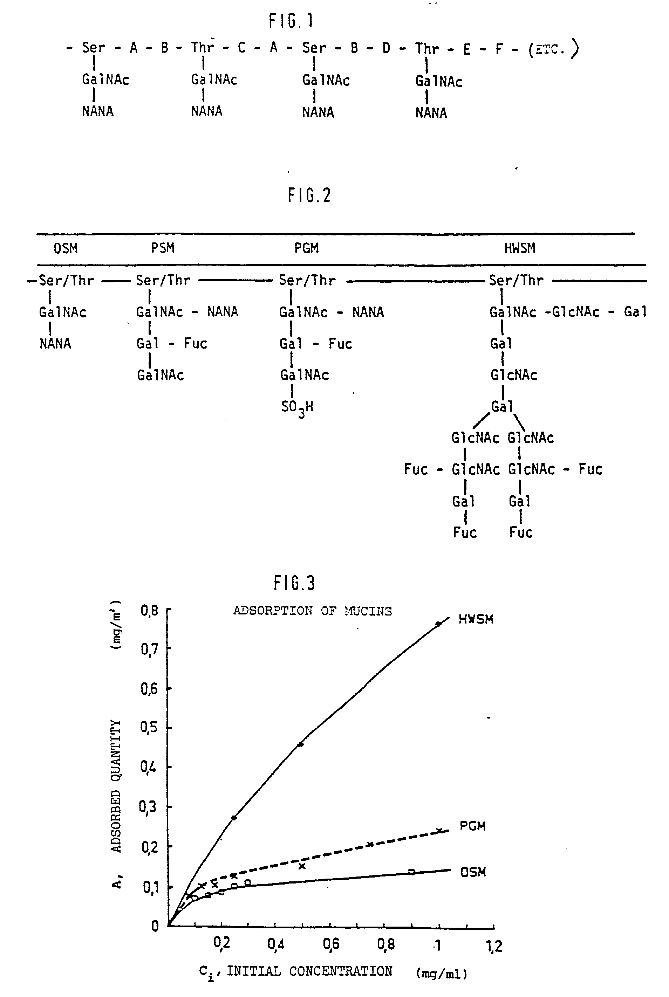

Construction of mucins
A mucin consists of a long, stretched polypeptide chain in which 1 in 3 to 4 amino acids is Threonine or Serine, to which a short carbohydrate chain may be bonded. The bonding sugar to these two hydroxy amino acids is always N-acetylgalactosamine (GalNac). The carbohydrate chains determine the type of mucin. One more (terminal) sugar may be bonded to the GalNac e.g. sialic acid (NANA) in submandibular mucin of sheep (OSM), but there may be more sugars, such as in HWSM.
An example of a model of OSM is shown in Figure 1.
The carbohydrate chains are mostly more complex of composition. Figure 2 shows diagrammatically a number of characteristic oligosaccharide chains of several mucins. The abbreviations used have the following meanings:
OSM, ovine submandibular mucin: disaccharide
PSM, porcine submandibular mucin: pentasaccharide
PGM, porcine gastric mucin, pentasaccharide with sulphate
HWSM, human whole salivary mucins. Figure 2 shows an example of a complex chain; there are other complex oligosaccharide chains with terminal sialic acid and also with sulphate-linked sugars.
For a considerable part, the carbohydrate chains determine the specific physico-chemical and biochemical properties of a mucin, such as its contribution to viscosity, adherence to dental surface, aggregation of bacteria etc. Table C gives a survey of the chemical composition of some mucins.

TABLE C
Chemical composition of mucins (% dry weight)

|  | Protein | Sialic acid | Fucose | Sulphate | Phosphate |
|---|---|---|---|---|---|
| PSM | 19.4 | 14.0 | 6.0 | — | — |
| OSM | 20.6 | 22.4 | — | — | — |
| PGM | 21.1 | 1.0 | 5.5 | 1.0 | — |
| HWSM | 18.2 | 5.1 | 17.4 | 1.4 | 0.14 |

In addition to there being a difference in the chemical composition of mucins, this also applies to their spacial structure. There are mucins built up from one polypeptide chain (e.g. OSM), but there are also those which via S—S bridges between Cysteine residus of the polypeptide chain are linked together to form a larger complex, e.g. PGM. This mucin is built up from 4 sub-units of 500,000 dalton each, with a total molecular weight of $2\times10^6$ dalton. The spacial structure of a mucin also has an effect on the physico-chemical properties, in particular the viscosity.

Quantitative data of adsorption of HWSM to hydroxyapatite
The adsorption of HWSM to hydroxyapatite, as a model for dental enamel was carried out as follows:
— 10 mg hydroxyapatite (HAP) of Biorad (specific area 72.5 $m^2$ g. is washed 3 times with distilled water
— adsorbate (HWSM) is dissolved in 2 mM K phosphate buffer (pH 6.3 or 7.0) and added to HAP
— incubation at room temperature for 4 hours in closed tubes by continuously rotating these at 6 revolutions per minute.
— after termination of the incubation, HAP is immediately sedimented by means of centrifugation.
— the amount of adsorbed HWSM is determined by means of protein and sialic acid assay in the supernatant.
From these data the maximum binding capacity of HWSM (N value in µg adsorbates/$m^2$ HAP) can be calculated and graphically determined by means of the Langmuir's adsorption equation:

$$\frac{C_p}{A} = \frac{C_p}{N} + \frac{1}{K.N}$$

In which:
$C_p$=equilibrium concentration of the adsorbate (mg/ml)
A=adsorbed quantity (mg/$m^2$)
N=maximum binding capacity (mg/$m^2$)
K=affinity constant (ml/mg)
In Table D the N-values of the several mucins are tabulated.

TABLE D
Maximum binding capacity of mucins at pH 6.3

|  | N (µg dry weight/$m^2$ HAP) |
|---|---|
| PSM | 72 |
| OSM | 113 |
| PGM | 259 |
| HWSM | 2.640 |

By way of illustration the adsorption of OSM, PGM and HWSM is shown in Figure 3.
The human salivary mucins are adsorbed extremely strongly to hydroxy apatite as compared with animal mucins. The maximum binding capacity (N) of HWSM is 2640 µg/$m^2$ HAP. The tested salivary mucins of animal origin (porcine, PSM; ovine OSM; bovine BSM;), however, have a 10—20 fold lower maximum binding capacity. On the other hand, the parotidal saliva also contains proteins which have a comparable maximum binding capacity to HWSM, e.g. Statherine and Proline-rich proteins. The proteins of parotidal saliva, however, do not protect dental enamel against the action of acid, which HWSM does.

7

EP 0 174 704 B1

As saliva contains many kinds of proteins which each can adsorb separately to HAP in partiuclar phosphate-containing proteins, it has been examined whether there is any competition when two components are simultaneously incubated with HAP. As a model for a phosphate component, we selected phytate which exhibits a strong bond to HAP (N=700 µg/m² HAP). Phytate is hexakis-phosphate of myoinositol which can be isolated from grains. It has a molecular weight of 924.

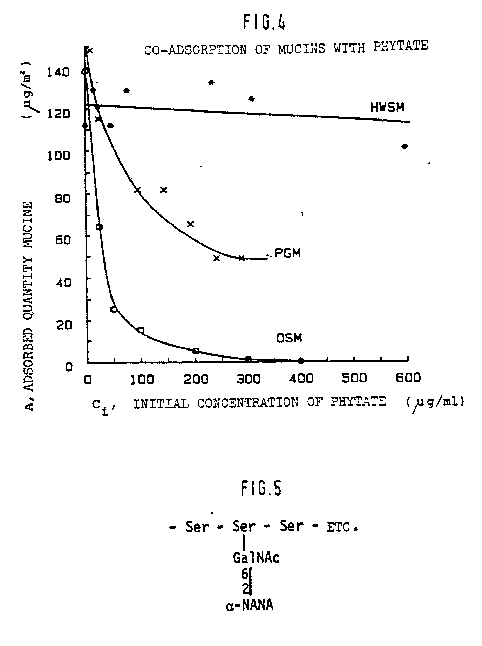

Phytate is capable of preventing the adsorption of OSM, even in very low concentrations. When 300 µg OSM is incubated with HAP, 30 µg phytate will reduce the adsorption of OSM to a value as low as 50%, while 300 µg phytate will completely prevent the adsorption of OSM (Figure 4).

The adsorption of PGM can be reduced to 30% by phytate but the inhibition of the adsorption is much slower than in the case of OSM. On the other hand phytate has only a minute effect or none at all on the adsorption of HWSM. Even at high phytate concentration (600 µg) the decrease in adsorption of HWSM is only very small. Apparently, HWSM attaches much more strongly to HAP than do OSM and PGM, which is in agreement with the N values calculated.

The reason for the difference in adsorption behaviour could be the difference in chemical composition and in functional groups and in the conformation of the molecules.

Table E gives a survey of the number of phosphate, sulphate and sialic acid groups per molecule of 500,000 dalton of the mucins tested.

TABLE E
Number of negatively charged groups of the mucins on the
basis of a molecular weight of 500,000

| Number of groups per molecule | | | | |
|---|---|---|---|---|
| | PSM | OSM | PGM | HWSM |
| Phosphate (-$PO_3H_2$) | — | — | — | 7 |
| Sulphate (-$SO_3H$) | — | — | 62 | 88 |
| Sialic acid (-COOH) | 227 | 362 | 16 | 82 |

As shown, PSM and OSM have a very large number of sialic acid groups, but no sulphate and phosphate. PGM has a small number of sialic acid groups and carries 62 sulphate groups. On the other hand, HWSM has a modest number of sialic acid groups but in addition the highest numer of sulphate groups and in addition 7 phosphate groups. Possibly the combination of sialic acid-sulphate-phosphate results in the extraordinarily strong adsorption of HWSM to HAP.

Protection of teeth against decalcification

Using two different methods, it has been tested whether HWSM can prevent the decalcification (dimineralisation) of teeth:

— when HWSM is adsorbed to HAP, only little $Ca^{2+}$ of the mineral is dissolved. With phosphate-containing compounds, such as phytate, however, much more $Ca^{2+}$ is dissolved as will become apparent from the following Table F.

TABLE F
Dissolving $Ca^{2+}$ from hydroxyapatite at pH 6.3

| | $Ca^{2+}$ (µmole/ml) |
|---|---|
| HWSM | 0.4 |
| HWSM+Phytate | 1.0 |
| Phytate | 1.7 |

— human and bovine dental enamel, after being embedded and polished were incubated with:
a. SM-SL saliva, dialysed
b. SM-SL saliva, dialysed, thereafter addition of $Ca^{2+}$ to 1 mM
c. Parotidal saliva, without dialysis, with and without $Ca^{2+}$ addition.

Incubation took place for 7 days with 20 ml saliva/tooth. The salivas were refreshed every day. Subsequently, the teeth were incubated in 1% citric acid solution for 1 minute to effect decalcification. This can be assayed by surface measurement. It is only SM-SL saliva to which, after dialysis, 1 mM $Ca^{2+}$ has

been added, which gives a protection against decalcification up to 100%. Apparently $Ca^{2+}$ is essential to prevent decalcification.

In addition, comparable experiments have been conducted with 0.1% HWSM solution in 1 mM $Ca^{2+}$, pH 7.0. From as short as 30 minutes' incubation, clear inhibition of the decalcification can be demonstrated, which increases to 100% in the time.

These experiments show that the adsorption of proteins to dental enamel need not necessarily mean that the teeth are also protected against decalcification. Parotidal proteins are adsorbed, but do not prevent decalcification, unlike HWSM.

Aggregation of oral micro-organisms by HWSM

Saliva contains various high-molecular proteins which are capable of aggregating various types of oral micro-organisms, so that these are no longer able to colonize. After aggregation, these microorganisms are passed on to the alimentary canal.

One of these high-molecular saliva components has been found to be HWSM. Human salivary mucins in low concentration are already capable of causing various types of oral micro-organisms to aggregate.

Synthesis of mucin-like biopolymers in vitro.

In order to produce sufficient quantities of commercially available mucins for their use in toothpaste and artificial saliva, it is desirable for them to be artificially synthesised. As the construction of a mucin is relatively simple, this is a realistic possibility. For this purpose poly-Ser and poly-Thr, consisting of chains of 500 to 4000 amino acids, with molecular weights ranging from 50,000 to 400,000 can be used as starting materials for the polypeptide chain. Subsequently the binding sugar GalNac is coupled to the Ser and Thr. This can be done either by means of an organic chemical reaction or by means of an enzymatic reaction using GalNac-transferase.

A suitable GalNac-transferase reaction mixture is:
1.3 mg serum albumin
0.29 nmol UDP-GalNac
0.375 μmol dithiothreitol
1.25 μmol $MnCl_2$
0.5 mg poly-Ser of poly-Thr
7.5 μmol HEPES buffer, pH 6,8
The total volume of the reaction mixture is 125 μl. The incubation is carried out at 37°C for 60 minutes. (Ko and Raghupathy, Biochem, Biophys. Res, Comm. *46*, (1972) 1704).

Subsequently, a sialic acid residue is coupled to GalNac by means of a specific enzyme sialyl transferase, which is especially present in the salivary glands.

A suitable sialyl transferase reaction mixture is:
1 mg poly-Ser-GalNac or poly-Thr-GalNac
1 mg sialyl transferase from salivary glands
800 μg Bovine Serum Albumin (BSA)
80 μmol Tris-maleate buffer pH 6.8
2.9 μmol CMP-NANA
Incubation takes place at 37°C for 3—6 hours. (Van den Eijnden et al., Biochem, Biophys. Res. Comm. *92* (1980) 839—845).

By means of this reaction, sialic acid can be bonded to GalNac via an α (2—6) bond. An example of the product thus obtained is shown in Figure 5.

If desired, variations can be made in the sugar chain. The advantage of such an artificial mucin is that it will be biologically degradable as it contains natural peptide and glycosidic bonds.

Uses

I. Toothpaste

The proposed mucins, which contribute to the protection of dental enamal against decalcification can be added to a toothpaste. Of essential importance for the activity of mucins is that they are not bonded to the polishing agent (often $CaCO_3$) of the toothpaste. It has surprisingly been found that they satisfy this condition.

A toothpaste that has been found to be suitable has the following composition (in parts by weight):

| 23 | glycerol |
| 32 | water |
| 40 | polishing agent ($CaCO_3$) |
| 1.4 | Na carboxymethyl cellulose |
| 0.15 | Na saccharine |
| 0.5 | Na fluoride |
| 1 | Na-dodecyl-sulphate |
| 0.1 | formalin |
| 0.8 | menthol |
| 0.1—0.5 | mucin |

Such a toothpaste, which contains HWSM or synthetic analogues on the basis of poly-Ser or poly-Thr in low concentration, has been found to effectively prevent the decalcification of the teeth owing to the action of acid. In addition, HWSM is capable of aggregating various types of oral micro-organisms, thus preventing their colonization. The result is that less acid is produced in the oral cavity.

II. Artificial saliva

The proposed mucins can be added to an artificial saliva to give xerostomia patients a better protection against the decalcification of their teeth and to protect the oral epithelial tissue from drying-up, inflammation and infections.

An artificial saliva that has been found to be suitable has the following composition (in g/100 ml):

| | |
| --- | --- |
| human salivary mucins/ artificial mucins | 1—2 |
| serum albumin | 2—4 |
| KCl | 0.12 |
| NaCl | 0.085 |
| $MgCl_2$ | 0.005 |
| $K_2HPO_4$ | 0.035 |
| K-thiocyanate | 0.01 |
| Na fluoride | 0.005 |
| menthol | 0.05 |
| pH | 7.0 |

The decalcification process of the teeth of xerostomia patients usually proceeds at an extremely high rate, and constitutes a major problem. In addition, the dry-mouth phenomenon causes painful irritation of the oral epithelial tissue and also gives speech and swallowing difficulties.

The addition of HWSM or synthetic analogues in low concentration to artificial saliva offers the following advantages:

— HWSM binds extremely strongly to dental enamel and offers a better protection of the teeth against decalcification owing to the action of acid.

— HWSM aggregates certain oral micro-organisms, thus preventing their colonization in the oral cavity. In this way, the harmful acid production of these organisms with anaerobic fermentation is indirectly prevented.

**Claims**

1. A composition for protecting teeth against acid-action-induced decalcification, characterised by comprising mucins isolated from human mucous salivary glands, or synthetic analogues of human mucins.

2. A composition as claimed in claim 1, characterised in that it is a toothpaste.

3. A composition as claimed in claim 1, characterised in that it is an artificial saliva.

4. A composition as claimed in any of claims 1—3, characterised in that the mucins or synthetic analogues thereof have a molecular weight of 0.5—2 megadalton.

5. A composition as claimed in any of claims 1—4, characterised in that the mucins or synthetic analogues thereof consist as to 10—30% by weight of protein and as to 70—90% by weight of sugars.

6. A composition as claimed in any of claims 1—5, characterised in that the protein moiety of the mucins or synthetic analogues thereof consists of a polypeptide chain of 500—4000 amino acids.

7. A composition as claimed in any of claims 1—6, characterised in that the sugars are covalently bonded to the threonine and/or serine amino acids in the protein moiety of the mucins or synthetic analogues thereof by means of the binding sugar N-acetylgalactosamine (GalNac).

8. A composition as claimed in any of claims 1—7, characterised in that the sugar moiety of the mucins or synthetic analogues thereof comprises sialic acid and/or fucose.

9. A composition as claimed in any of claims 1—8, characterised in that the mucins or synthetic analogues thereof contain sulphate and/or phosphate groups.

10. A composition as claimed in any of claims 1—9, characterised by comprising synthetic analogues of human mucins, the protein moiety of which is formed by (homo)poly-Ser or (homo)poly-Thr, and the sugar moiety of which comprises one or more sugars from the group consisting of sialic acid, fucose, hexoses and hexosamines.

11. A composition as claimed in claim 10, characterised in that the synthetic analogues of human mucins contain sulphate and/or phosphate groups.

**Patentansprüche**

1. Zusammensetzung zum Schutz von Zähnen gegen säureinduzierte Entkalkung, gekennzeichnet durch den Gehalt von aus Human-Speicheldrüsen isolierten Muzinen oder synthetischen Analogen von Human-Muzinen.

2. Zusammensetzung nach Anspruch 1, dadurch gekennzeichnet, daß es sich um eine Zahnpasta handelt.

3. Zusammensetzung nach Anspruch 1, dadurch gekennzeichnet, daß es sich um einen künstlichen Speichel handelt.

4. Zusammensetzung nach einem der Ansprüche 1 bis 3, dadurch gekennzeichnet, daß die Muzine oder synthetischen Analogen hiervor ein Molekulargewicht von 0,5 bis 2 Megadalton besitzen.

5. Zusammensetzung nach einem der Ansprüche 1 bis 4, dadurch gekennzeichnet, daß die Muzine oder synthetischen Analogen hiervon zu 10 bis 30 Gewichts-% aus Protein und zu 70 bis 90 Gewichts-% aus Zuckern bestehen.

6. Zusammensetzung nach einem der Ansprüche 1 bis 5, dadurch gekennzeichnet, daß der Proteinrest der Musine oder synthetischen Analogen hiervon aus einer Polypeptidkette mit 500 bis 4000 Aminosäuren besteht.

7. Zusammensetzung nach einem der Ansprüche 1 bis 6, dadurch gekennzeichnet, daß die Zucker kovalent an die Threonin- und/oder Serin-Aminosäuren im Proteinrest der Muzine oder synthetischen Analogen hiervon mittels des verknüpfenden Zuckers N-Acetylgalactosamin (GalNac) gebunden sind.

8. Zusammensetzung nach einem der Ansprüche 1 bis 7, dadurch gekennzeichnet, daß der Zuckerrest der Muzine oder synthetischen Analogen hiervon Sialsäure und/oder Fukose enthält.

9. Zusammensetzung nach einem der Ansprüche 1 bis 8, dadurch gekennzeichnet, daß die Muzine oder synthetischen Analogen hiervon Sulfat- und/oder Phosphatgruppen enthalten.

10. Zusammensetzung nach einem der Ansprüche 1 bis 9, gekennzeichnet durch den Gehalt an synthetischen Analogen von Humanmuzinen, deren Proteinrest durch (Homo)Poly-Ser oder (Homo)Poly-Thr gebildet ist, und deren Zuckerrest einen oder mehrere Zucker aus der Gruppe Sialsäure, Fukose, Hexosen und Hexosamine enthält.

11. Zusammensetzung nach Anspruch 10, dadurch gekennzeichnet, daß die synthetischen Analogen der Humanmuzine Sulfat- und/oder Phosphatgruppen enthalten.

**Revendications**

1. Composition pour protéger les dents contre une décalcification provoquée par l'action d'acides, caractérisée en ce qu'elle contient des mucines isolées des glandes salivaires muqueuses humaines ou des analogues synthétiques de mucines humaines.

2. Composition selon la revendication 1, caractérisée en ce que c'est une pâte dentifrice.

3. Composition selon la revendication 1, caractérisée en ce que c'est une salive artificielle.

4. Composition selon l'une quelconque des revendications 1 à 3, caractérisée en ce que les mucines ou leurs analogues synthétiques ont un poids moléculaire de 0,5 à 2 mégadalton.

5. Composition selon l'une quelconque des revendications 1 à 4, caractérisée en ce que les mucines ou leurs analogues synthétiques contiennent 10 à 30% en poids de protéines et 70 à 90% en poids de sucres.

6. Composition selon l'une quelconque des revendications 1 à 5, caractérisée en ce que la partie protéines des mucines ou de leurs analogues synthétiques est constituée par une chaîne de polypeptides contenant 500 à 4000 acides aminés.

7. Composition selon l'une quelconque des revendications 1 à 6, caractérisée en ce que les sucres sont liés de façon covalente aux acides aminés: thréonine et/ou sérine, dans la partie protéines des mucines ou de leurs analogues synthétiques au moyen du sucre liant, N-acéylgalactosamine (GalNac).

8. Composition selon l'une quelconque des revendications 1 à 7, caractérisée en ce que la partie sucres des mucines ou de leurs analogues synthétiques est constituée par l'acide sialique et/ou le fucose.

9. Composition selon l'une quelconque des revendications 1 à 8, caractérisée en ce que les mucines ou leurs analogues synthétiques contiennent des groupes sulfates et/ou phosphates.

10. Composition selon l'une quelconque des revendications 1 à 9, caractérisée en ce qu'elle comprend des analogues synthétiques de mucines humaines, dont la partie protéines est formée par (homo) poly-Ser ou (homo) poly-Thr et dont la partie sucres comporte un ou plusieurs sucres dans le groupe constitué par l'acide sialique, le fucose, les hexoses et les hexosamines.

11. Composition selon la revendication 10, caractérisée en ce que les analogues synthétiques des mucines humaines contiennent des groupes sulfates et/ou phosphates.

11

## FIG. 1

- Ser - A - B - Thr - C - A - Ser - B - D - Thr - E - F - (ETC. )
   |             |             |            |
GalNAc     GalNAc     GalNAc     GalNAc
  |           |            |          |
NANA       NANA       NANA       NANA

## FIG.2

| OSM | PSM | PGM | HWSM |
|---|---|---|---|
| —Ser/Thr | — Ser/Thr | — Ser/Thr | — Ser/Thr — |
| GalNAc | GalNAc – NANA | GalNAc – NANA | GalNAc –GlcNAc – Gal |
| NANA | Gal – Fuc | Gal – Fuc | Gal |
| | GalNAc | GalNAc | GlcNAc |
| | | SO_3H | Gal |

HWSM (continued):

```
         Gal
       /    \
  GlcNAc    GlcNAc
     |          |
Fuc - GlcNAc  GlcNAc - Fuc
     |          |
    Gal        Gal
     |          |
    Fuc        Fuc
```

## FIG.3

ADSORPTION OF MUCINS

$A$, ADSORBED QUANTITY $(mg/m^2)$ vs $c_i$, INITIAL CONCENTRATION $(mg/ml)$

Curves labeled HWSM, PGM, OSM

## FIG.4

CO-ADSORPTION OF MUCINS WITH PHYTATE

A, ADSORBED QUANTITY MUCINE ($\mu g/m^2$)

HWSM

PGM

OSM

$c_i$, INITIAL CONCENTRATION OF PHYTATE ($\mu g/ml$)

## FIG.5

- Ser - Ser - Ser - ETC.
  |
  GalNAc
  6|
  2|
  α-NANA

2